# EUROPÄISCHE PATENTANMELDUNG

(11) **EP 1 430 900 A1**
(43) Veröffentlichungstag der Anmeldung: **23.06.2004**
(21) Anmeldenummer: 02293130.7
(22) Anmeldetag: 18.12.2002
(51) Int. Cl.: A61K 35/78

(54) **Verwendung eines Extraktes aus der Pflanze Argania spinosa**

(71) Anmelder: Cognis France S.A., 31360 Saint-Martory (FR)
(72) Erfinder: Henry, Florence, 54600 Villers-les-Nancy (FR); Danoux, Louis, 54420 Saulxures-les-Nancy (FR); Pauly, Gilles, 5400 Nancy (FR); Charrouf, Zoubida, Rabat R.P. (MA)
(74) Vertreter: Cabinet HERRBURGER

(57) **Zusammenfassung**

Vorgeschlagen wird die Verwendung von Extrakten aus der Pflanze Argania spinosa zur Herstellung von Anti-Akne-Mitteln, Zubereitungen gegen Seborrhoe und Mitteln anti-5-alpha-reductase-Aktivität, wobei der verwendete Extrakt Proteine und Saponine, insbesondere Arganin A enthält.

## Beschreibung

### Gebiet der Erfindung

Die Erfindung befindet sich auf dem Gebiet der kosmetischen und/oder dermopharmazeutischen Pflegestoffe und betrifft die Verwendung von Extrakten der Pflanze Argania spinosa zur Herstellung von Mitteln gegen Akne und/oder Seborrhoe, sowie Zubereitungen mit anti-5-alpha-Reductase-Aktivität.

### Stand der Technik

Fettige und durch Akne befallene Haut zeigt eine erhöhte Sekretion von Hautfett und Talg durch die übermäßige Aktivität der Talgdrüsen. Die in den Ausscheidungen der Talgdrüsen enthaltenen Triglyceride werden auf der Haut durch Lipasen verschiedener Microorganismen wie beispielsweise Corynebacterium acnes, Staphylococcus epidermidis und Pytirosporum ovale zersetzt und freie Fettsäuren werden abgespalten. Einige dieser freien Fettsäuren führen zu den charakteristischen entzündlichen Phänomenen des akuten Akne-Stadiums.

Die Transformation von Teststeron in 5-dihydrotestosterone (5-DHT) durch das Enzym 5-alpha-Reduktase hat sich als eine der Ursachen für die vermehrte Talgdrüsensekretion herausgestellt. Daher ist die Aktivität des Enzymes 5-alpha-Reduktase, das insbesondere in den Talgdrüsen und in apokrinen Drüsen, sowie in Keratinocyten und Fibroblasten nachzuweisen ist, von besonderer Wichtigkeit für die Haut, die durch Akne oder Seborrhoe ausgezeichnet ist.

Kosmetische Zubereitungen stehen dem Verbraucher heute in einer Vielzahl von Kombinationen zur Verfügung. Dennoch besteht im Markt das Bedürfnis nach Produkten mit einem verbesserten Leistungsspektrum. Hierbei sind Hautverträglichkeit sowie der Einsatz natürlicher Produkte beim Kunden gefragt. Daneben ist es wünschenswert, durch Kombination bereits bekannter Wirkstoffe, oder durch auffinden neuer Einsatzgebiete bereits bekannter Substanzklassen deutlich bessere Produkte zu erhalten. Besonders Extrakte von Pflanzen und deren Inhaltstoffe finden immer häufiger Einsatz in der Kosmetik und Pharmazie. Es sind jedoch viele Pflanzen und ihre potentielle Wirkung noch nicht gefunden worden und viele neue Anwendungsgebiete bereits bekannter Substanzklassen sind immer wieder überraschend.

Seit langem ist bekannt, dass viele Saponine, die aus den unterschiedlichsten Pflanzen und Mikroorganismen gewonnen werden, eine anti-radikalische, analgetische und auch antiinflammatorische Wirkung zeigen. Diese Wirkung konnten Alaoui et al. auch für die aus Argania spinosa isolierten Saponine nachweisen [Alaoui K. et al.; *Annales pharmaceutiques françaises*, **1998,** *56*, 220-228.]. Es wurde außerdem für einige Saponine eine antibiotische und eine fungistatische Wirksamkeit gefunden. Saponine, speziell die Triterpen-Saponine sind aus einem tetra- oder pentacyclischen Triterpen-Aglykon und einer oder zwei glycosidisch gebundenen Zuckerketten aufgebaut.

Die Aufgabe der vorliegenden Patentanmeldung hat darin bestanden, neue Anwendungen für gut verträgliche wirkstoffreiche Extrakte aus nachwachsenden pflanzlichen Rohstoffen zu finden, wobei insbesondere Aktivstoffe zur Behandlung Akne-befallener und seborrhoeischer Haut gesucht wurden.

### Beschreibung der Erfindung

Gegenstand der Erfindung ist die Verwendung von Extrakten aus der Pflanze Argania spinosa zur Herstellung von Anti-Akne-Mitteln, zur Herstellung von Zubereitungen gegen Seborrhoe, sowie zur Herstellung von Zubereitungen mit anti-5-alpha-reductase-Aktivität. Überraschenderweise wurde gefunden, dass durch den Einsatz von Extrakten der Pflanze Argania spinosa Zubereitungen hergestellt werden können, die eine hervorragende Wirkung in Anti-Akne-Mitteln und Zubereitungen gegen Seborrhoe aufweisen und gleichzeitig eine hohe Hautverträglichkeit besitzen. Sie sind daher hervorragend gegen fettige Haut, Akne-Haut und fettige Kopfhaut auch bei sensiblen Hauttypen einzusetzen. Die hergestellten Zubereitungen zeigen eine deutliche anti-5-alpha-Reductase-Aktivität.

### Argania spinosa

Die erfindungsgemäß einzusetzenden Extrakte werden aus Pflanzen der Familie der Sapotaceae, speziell aus Argania spinosa gewonnen. Bei dieser Pflanze handelt es sich um einen an den Ölbaum erinnernden Baum, der überwiegend in Marokko an der Westseite des Atlasgebirges zu finden ist. Er bildet an seinen knorrigen Ästen und bedornten Zweigen Beeren von der Größe und Gestalt der Oliven mit ein bis zwei Samenkernen. Das nussartig schmeckende Öl aus den Samenkernen dient unter anderem als Speiseöl.

Unter dem Begriff Pflanze im Sinne der vorliegenden Anmeldung sind sowohl ganze Pflanzen als auch Pflanzenteile (Blätter, Wurzeln, Stamm, Rinde, Blüten, Früchte, Fruchtfleisch und Samenkerne) sowie deren Gemische zu verstehen. Besonders bevorzugt zur Extraktion der Saponine im Sinne der Erfindung sind die Samenkerne der Frucht dieser Pflanze insbesondere die Extraktion des Rückstands aus den entfetteten Samenkernen.

### Saponine

Unter Saponinen sind im Sinne der Erfindung grundsätzlich alle Saponine zu verstehen, die sich aus der Pflanze Argania spinosa isolieren lassen.

Aus dem Rückstand, der bei der Ölgewinnung aus den Samenkernen von Argania spinosa anfällt, werden Saponine erhalten, die sich in der Struktur von Saponinen aus anderen Pflanzen unterscheiden [Charrouf Z., et al.; *Phytochemistry*, **1992,** *31*; 2079-2086.]. Es handelt sich hier um Saponine mit der Bezeichnung Arganin A, Arganin B, Arganin C, Arganin D, Arganin E, Arganin F und Misaponin A. Aus dem Stamm der Pflanze können die einsetzbaren Saponine Arganin G, Arganin H, und Arganin J [Oulad-Ali A., et al.; *J. Nat*. *Prod*.; **1996,** *59*, 193-195.]. Das Aglycon dieser Saponine weist die im Folgenden dargestellte Struktur (I) auf, die genannten Saponine unterscheiden sich jeweils in den Zuckereinheiten an R1 und R3 bzw. durch eine Hydroxygruppe an R2. Bei R3 handelt es sich um ein Tetrasaccharid und bei R1 jeweils um ein Mono- oder Disaccharid (z. B. 1,6-Diglukose für Arganin A und B).

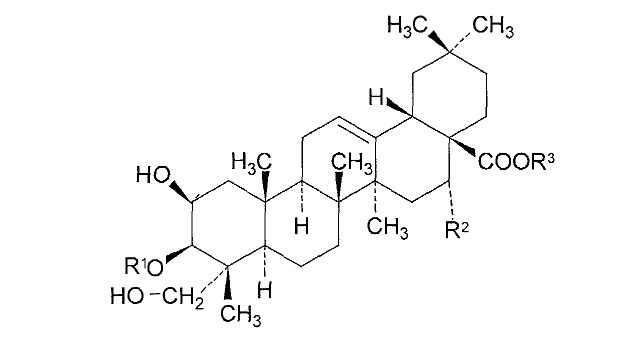

Die erfindungsgemäßen Saponine zeigen in toxikologischen Test an Mäusen und Ratten eine geringe Toxizität. Im Vergleich zu anderen Saponinen wie z.B. aus Gypsophila paniculata konnten die Erfinder auch durch Tests an humanen Fibroblasten eine wesentlich geringere Toxizität nachweisen.

Die erfindungsgemäß einzusetzenden Saponine entsprechen Arganin A, Arganin B, Arganin C, Arganin C, Arganin D, Arganin E, Arganin F, Misaponin A, sowie Arganin G, Arganin H, und Arganin J. Sie können als Gemisch von zwei oder mehr, oder als Reinsubstanz in der kosmetischen und oder pharmazeutischen Zubereitung Anwendung finden. Besonders bevorzugt sind Mischungen aus Arganin A, Arganin B, Arganin C, Arganin C, Arganin D, Arganin E, Arganin F, Misaponin A, wobei die Anteile der Saponine in den Mischungen variieren können. Vorzugsweise werden Extrakte eingesetzt, die eine hohe Menge an Arganin A enthalten. Die erfindungsgemäße Verwendung von Extrakten mit mindestens 6 Gew. %, vorzugsweise 8 Gew.% und insbesondere mindestens 10 Gew. % an Arganin A - bezogen auf das Tockengewicht des Extraktes - haben sich durch ihre besonders ausgeprägten Wirkungen ausgezeichnet.

### Proteine

Unter Proteinen sind im Sinne der Erfindung solche zu verstehen, die sich aus der Pflanze Argania spinosa isolieren lassen. Bevorzugt ist die Extraktion der Samenkerne, insbesondere der entfetteten Samenkerne nach der Ölextraktion. Dementsprechend ist eine besondere Ausführungsform der Erfindung Zubereitungen, die native Proteine enthalten, die erhalten werden aus einem Extrakt der Samenkerne, insbesondere der entfetteten Samenkerne von Argania spinosa.

Unter der bevorzugten Extraktion der entfetteten Samenkerne ist im Sinne der Erfindung zu verstehen, dass bevorzugt der Rückstand - eine Art Kuchen - aus der Extraktion zur Ölgewinnung aus den Samenkernen von Argania spinosa extrahiert wird. Dieser bevorzugt zu extrahierende Rückstand aus der Extraktion zur Ölgewinnung enthält 3 bis 10 Gew.-% restliches Öl. Aus diesem Rückstand werden die erfindungsgemäßen Proteine vom noch verbliebenen Öl möglichst vollständig getrennt. Neben Proteinen können noch weitere, natürlich in den Pflanzen Argania spinosa vorkommende Substanzen mit extrahiert werden, die unter den gleichen Bedingungen extrahierbar sind.

In einer weiteren Ausführungsform der Erfindung enthalten die erfindungsgemäßen Zubereitungen native Proteine, die durch wässrige Extraktion bei einem pH-Wert geringer oder gleich 12, bevorzugt zwischen 3,5 und 6,5, insbesondere entweder zwischen 5,5 und 6,5 oder zwischen 3,5 und 5,5 und gegebenenfalls einer anschließenden Trocknung, beispielsweise einer Sprüh- oder Gefriertrocknung erhalten werden. Der gewählte pH-Wert Bereich ist abhängig von der zu isolierenden Proteinfraktion.

Die nativen Proteine, die sich aus der Pflanze Argania spinosa, insbesondere aus den Samenkernen der Pflanze extrahieren lassen, können Molekulargewichte zwischen 10.000 Da und größer als 500.000 Da besitzen. Bevorzugt können sie eingeteilt werden in folgende Gruppen von Molekulargewichtsbereichen. Extrahiert werden können native Proteine mit einem Molekulargewicht größer als 500.000 Da, native Proteine mit Molekulargewicht im Bereich von 170.000 bis 250.000 Da und native Proteine mit Molekulargewicht im Bereich von 10.000 bis 18.000 Da.

Demnach betreffen weitere Ausführungsformen der Erfindung zum einen Zubereitungen, die native Proteine enthalten, deren Molekulargewicht größer als 500.000 Da beträgt, Zubereitungen, die native Proteine enthalten, deren Molekulargewicht im Bereich von 170.000 Da bis 250.000 Da, bevorzugt im Bereich von 170.000 Da bis 210.000 Da liegt und Zubereitungen, die native Proteine enthalten, deren Molekulargewicht im Bereich von 10.000 bis 18.000 bevorzugt im Bereich von 13.000 bis 16.000 liegt.

Bevorzugt soll der Anteil an Proteinen im erfindungsmäßig eingesetzten Extrakt mindestens 3 Gew. %, vorzugsweise mindestens 4 Gew. % und insbesondere mindestens 5 Gew. % - bezogen auf das Trockengewicht des Extraktes - betragen

### Extraktion

Die Herstellung der erfindungsgemäß einzusetzenden Extrakte erfolgt durch übliche Methoden der Extraktion von Pflanzen bzw. Pflanzenteilen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf Hagers Handbuch der Pharmazeutischen Praxis, (5. Auflage, Bd. 2, S. 1026-1030, Springer Verlag, Berlin-Heidelberg-New-York 1991) verwiesen. Als Ausgangsmaterial können frische oder getrocknete Pflanzen oder Pflanzenteile eingesetzt werden, üblicherweise wird jedoch von, entfetteten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Zerstoßung mit einem Mörser genannt. In einer besonderen Ausführungsform werden die verwendeten Extrakte erhalten durch Extraktion des Stammes, der Wurzel, der Blätter, der Blüte oder der Früchte. Besonders bevorzugt ist die Extraktion er Samenkerne.

Als Lösungsmittel für die Durchführung der Extraktionen können vorzugsweise organische Lösungsmittel, Wasser oder Gemische aus organischen Lösungsmitteln und Wasser, insbesondere niedermolekulare Alkohole, Ester, Ketone oder halogenhaltige Kohlenwasserstoffe mit mehr oder weniger hohen Wassergehalten (destilliert oder nicht destilliert) vorzugsweise wässrig, alkoholische Lösungen einer Temperatur von über 20 °C, (im nachfolgenden als Raumtemperatur bezeichnet), verwendet werden. Besonders bevorzugt ist die Extraktion mit Wasser, Methanol, Ethanol, Aceton, Propylenglycolen, Polyethylenglycolen Ethylacetat, Dichlormethan, Trichlormethan sowie Mischungen hieraus. Die Extraktion erfolgt in der Regel bei 20 bis 100 °C, bevorzugt bei 20 bis 85°C, insbesondere bei Raumtemperatur. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt. Typische Ausbeuten (=Trockensubstanzmenge des Extraktes bezogen auf eingesetzte Rohstoffmenge) bei der Extraktion getrockneter Pflanzen oder getrockneter Pflanzenteile gegebenenfalls entfettet, liegen im Bereich von 3 bis 20, insbesondere 4 bis 16 Gew.-%. Die vorliegende Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden.

Erfindungsgemäß enthalten die Extrakte dieser Pflanze 10 bis 99 Gew.-% Saponine, vorzugsweise 15 bis 70 Gew.-%. Die Einsatzmenge der Pflanzenextrakte in den Anti-Akne-Mitteln und Zubereitungen gegen Seborrhoe, sowie Zubereitungen mit anti-5-alphareductase-Aktivität richtet sich nach der Konzentration der einzelnen Inhaltstoffe. Die Gesamtmenge des Pflanzenextraktes, der in den Zubereitungen enthalten ist, beträgt in der Regel 0,01 bis 25 Gew.-%, vorzugsweise 0,03 bis 5 Gew.-%, insbesondere 0,03 bis 0,4 Gew.-% bezogen auf die Endzubereitung.

Vorzugsweise enthalten die eingesetzten Pflanzenextrakte proteine und Saponine in den beschriebenen Mengenbereichen in Kombination.

### Gewerbliche Anwendbarkeit

### Kosmetische und/oder pharmazeutische Zubereitungen

Die Verwendung von Extrakten aus der Pflanze Argania spinosa zur Herstellung von Anti-Akne-Mitteln, Zubereitungen gegen Seborrhoe, sowie Zubereitungen mit anti-5-alphareductase-Aktivität resultiert in kosmetischen und/oder pharmazeutischen Zubereitungen wie beispielsweise Cremes, Gelen, Lotionen, alkoholischen und wäßrig/alkoholischen Lösungen, Emulsionen, Haarshampoos, Haarlotionen, Schaumbädern, Duschbädern, Wachs/ Fett-Massen, Stiftpräparaten, Pudern oder Salben. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Filmbildner, Quellmittel, Hydrotrope, Solubilisatoren, Konservierungsmittel, Parfümöle, Farbstoffe und dergleichen enthalten. Der Gesamtanteil der Hilfs- und Zusatzstoffe kann 1 bis 70, vorzugsweise 20 bis 50 und insbesondere 5 bis 40 Gew.-% - bezogen auf die Endzubereitung der kosmetischen und/oder pharmazeutischen Zubereitungen - betragen. Die Herstellung der Zubereitungen kann durch übliche Kalt - oder Heißprozesse erfolgen; vorzugsweise arbeitet man nach der Phaseninversionstemperatur-Methode.

### Beispiele

### Beispiel 1: Herstellung des Saponin-Rohextraktes

0.3 kg eines Argania spinosa Kuchens (aus den Samen nach der Ölextraktion) wurden mit 1,98 kg Hexan entfettet (1 Stunde bei 80°C). Der entfettete Kuchen wurde nachfolgend bei Raumtemperatur für 24 Stunden getrocknet.
0,12 kg des entfetteten und getrockneten Kuchens wurden in einem Rührgefäß mit 2 1 80 Vol.% Ethanol aufgefüllt. Diese Mischung wurde bei Raumtemperatur über 16 Stunden gerührt. Danach wurden die Feststoffe durch Filtration entfernt. Die gefilterte Lösung bildet den Rohextrakt aus dem Ethanol durch Evaporieren entfernt wurde. Abschließend wurde derRückstand durch Lyophilisation getrocknet.

### Prinzip des Tests

Rekonstruierte Epidermis enthält vergleichbar zur lebenden Haut den gesamten enzymatischen Mechanismus der zur Metabolisierung von Testosteron benötigt wird. Der Versuchsansatz ist weniger restriktiv als eine Untersuchung der Inhibition gereingigter 5-alpha-reductase, da Zwischenprodukte der Testosteronmetabolisierung untersucht werden und nicht 5-DHT, die ebenfalls in den Problemkreis der fettigen Haut eingreifen und da die Enzyme dieses Metabolismus unter physiologischen Bedingungen untersucht werden [Bernard F-X et al, 2000, Int. J. Cosmetic Science, 22, 397-407, Expression of type 5-alpha-reductase and metabolism of testosterone in reconstructed human epidermis - SkinEthic: a new model for screening skin targeted androgen modulators]

### Testaufbau:

### Material:

Epidermis SkinEthic (17 Tage, 0.63 cm²) in Kultur, 37°C, 5% CO2
Referenz - Substanz: Finasteride
Testosterone: [4-¹⁴C] Testosterone (Amersham, CFA129, 56 mCi/mmole), 250 nCi/Epidermis
Extrakt von Argania Saponinen gemäß Beispiel 1

### Behandlung:

Die Epidermis wurde vorkultiviert auf Platten mit 24 Positionen für 24 Stunden. Die Untersuchungen mit den Produkten und der Referenzsubstanz Finasterid (10 µM) wurden in dreifacher Ausführung durchgeführt (3 Epidermisansätze pro Experiment). Nach 24-stündiger Behandlung wurden die Medien der Subepidermis erneuert und durch 300 µl frisches Kulturmedium ausgewechselt. 100 µl radioaktiv markierter Testosteronlösung wuirde auf die Oberseite (Hornhaut) der Epidermis aufgetragen (TO). Nach weiteren 24 Stunden wurde das Subepidermis-Medium für die Analyse entnommen.
Die Lebensfähigkeit der Keratinocyten in den unterschiedlichen Epidermisproben wurde am Ende des Versuches mit der MTT-Methode untersucht.

### Extraktion und Analyse:

Um die transepidermale Diffusion zu bestimmen wurden je 20 µl jedes Kulturmediums entnommen und im Liquid Scintillationszähler gezählt.
Die im Kulturmedium enthaltenen Steroide wurden für die Analyse des Metabolismus extrahiert und dünnschichtchromatographisch (auf Kieselgel) in ihre molekularen Derivate getrennt. Die Menge an transformiertem Testosteron wurde durch radioaktive Zählung der unterschiedlichen Spots mit einem Phosphoimager ermittelt.

### Ergebnisse:

Die Überlebensfähigkeit der behandelten Epidermis und die transepidermale Diffusion sind in Tabelle 1 zusammengefaßt.

**Table 1:**

| **Diffusion von** ^{**14**}**C-Testosterone (und Metaboliten) durch rekonstruierte humane Epidermis (SkinEthic) und Überlebensfähigkeit des Gewebes am Ende des Versuches (t=24 Stunden)** | | | |
|---|---|---|---|
| **Behandlung** | **%** ^{**14**}**C-Testosterone** | **Nmole Steroid** | **Überlebensrate in %** |
| Total Testosterone | / | 4.5 | / |
| Kontrolle | 100 | 2.2 | 100 |
| Finasterid 10 µM | 110 | 2.4 | 101 |
| Extrakt gemäß Beispiel 1 Argania | | | |
| 0.003% | 98 | 2.2 | 98 |
| 0.001% | 100 | 2.2 | 101 |

Die Überlebensfähigkeit unbehandelter Epidermis (Kontrolle) und mit Finasterid behandelter Epidermis war identisch mit den durch Extrakt-behandelten Proben (2 Konzentrationen).

Für die transepidermale Diffusion wurde ungefähr ein Mittel der initialen Radioaktivität im Kulturmedium nach 24 Stunden Inkubation nachgewiesen. Die Behandlung mit Finasterid führte zu einem geringfügigen Anstieg der Diffusion von Steroiden durch die Epidermis (110% der Kontrolle).
Der untersuchte Extrakt jedoch hat die Diffusion von Steroiden durch die Epidermis nicht beeinflußt (Werte zwischen 98 und 100% der Kontrolle).

Die Metabolisierung von Testosteron ist in Tabelle 2 zusammengefaßt.

**Table 2:**

| **Einfluß der Behandlung mit Argania Extrakt auf die Produktion von DHT. Analyse durch Phosphorimager im Zusammenhang mit der angefallenen Radioaktivität** | |
|---|---|
| **Behandlung** | **Entstandenes DHT in %** |
| Kontrolle | 100 |
| Finasterid 10 µM | 8 |
| Extrakt von Argania Saponinen gemäß Beispiel 1 0.001% 0.003% | 61% 74% |

Der untersuchte Extrakt zeigt eine deutliche Verminderung der Produktion an DHT - 39 und 26 % Inhibierung. In einer Konzentration von 0,001 Ge. % ist bereits eine signifikante Inhibierung der 5-alpha-Reduktase Aktivität zu detektieren ohne dass dabei toxische Effekte die Überlebensfähigkeit der Zellen beeinflussen (bestimmt im MTT-Test).

## Patentansprüche

1. Verwendung von Extrakten aus der Pflanze Argania spinosa zur Herstellung von Anti-Akne-Mitteln.

2. Verwendung von Extrakten der Pflanze Argania spinosa zur Herstellung von Zubereitungen gegen Seborrhoe.

3. Verwendung von Extrakten der Pflanze Argania spinosa zur Herstellung von Zubereitungen mit anti-5-alpha-Reductase-Aktivität.

4. Verwendung nach mindestens einem der Ansprüche 1 bis 3, **dadurch gekennzeichnet, dass** die Extrakte Proteine und/oder Saponine enthalten.

5. Verwendung nach mindestens einem der Ansprüche 1 bis 4, **dadurch gekennzeichnet, dass** die Extrakte Saponine enthalten, die ausgewählt sind aus der Gruppe, die gebildet wird von Arganin A, Arganin B, Arganin C, Arganine D, Arganin E, Arganin F, Arganin G, Arganin H, Arganin J und Misaponin A.

6. Verwendung nach mindestens einem der Ansprüche 1 bis 5, **dadurch gekennzeichnet, dass** die Extrakte als Saponin Arganin A in Mengen von mindestens 6 Gew.% bezogen auf das Trockengewicht des Extraktes enthalten.

7. Verwendung nach mindestens einem der Ansprüche 1 bis 6, **dadurch gekennzeichnet, dass** die Extrakte mindestens 3 Gew.% an Proteinen bezogen auf das Trockengewicht des Extraktes enthalten.

8. Verwendung nach mindestens einem der Ansprüche 1 bis 7, **dadurch gekennzeichnet, dass** die Extrakte in Mengen von 0,01 bis 25 Gew.-% berechnet als Trockengewicht, bezogen auf die Mittel, eingesetzt werden.

9. Verwendung nach mindestens einem der Ansprüche 1 bis 8, **dadurch gekennzeichnet, dass** der Extrakt erhalten wird durch Extraktion von Pflanzenteilen, ausgewählt aus der Gruppe, die gebildet wird aus den Blättern, den Wurzeln, dem Stamm, der Rinde, den Blüten, den Früchten, dem Fruchtfleisch und den Samenkernen.

10. Verwendung nach mindestens einem der Ansprüche 1 bis 9, **dadurch gekennzeichnet, dass** der Extrakt erhalten wird durch Extraktion der Samenkerne und/oder der entfetteten Samenkerne aus der Frucht der Pflanze.
